# EUROPEAN PATENT APPLICATION

(11) **EP 1 810 607 A1**
(43) Date of publication of application: **25.07.2007**
(21) Application number: 05782010.2
(22) Date of filing: 09.09.2005
(51) Int. Cl.: A61B 1/00, A61B 5/07

(54) **RECEIVING DEVICE, AND SYSTEM FOR INTRODUCTION INTO SPECIMEN UNDER TEST**

(30) Priority: 09.09.2004 JP 2004263002
(71) Applicant: Olympus Optical Corporation Limited, Tokyo 151-0072 (JP)
(72) Inventor: SHIGEMORI, Toshiaki, Hachioji-shi, Tokyo 1920023 (JP)
(74) Representative: von Hellfeld, Axel
(86) International application number: PCT/JP2005/016651
(87) International publication number: WO 2006/028220

(57) **Abstract**

A receiving apparatus 3 includes a receiving circuit 22 which performs reception processing such as demodulation of radio signals received by one of receiving antennas 6a to 6h, an image processing unit 26 which reconfigures image data based on original signals obtained by the reception processing, and a timing controller 28 which controls timing of processing operations of the receiving circuit 22 and the image processing unit 26 so that the processing operations are not performed simultaneously. Since the processing operation of the receiving circuit 22 and the processing operation of the image processing unit 26 are controlled so as not to overlap with each other, noises generated by the processing operation of one element can be prevented from negatively affecting the processing operation of the other element. Thus, mutual interference between the operation of a receiving mechanism and the operation of a signal processing mechanism can be suppressed while an increase in size of the apparatus is prevented.

## Description

### TECHNICAL FIELD

The present invention relates to a receiving apparatus which performs reception processing of radio signals transmitted intermittently, and a body insertable system including the receiving apparatus.

### BACKGROUND ART

In recent years, a swallowable capsule endoscope has been proposed in a field of endoscope. The capsule endoscope is equipped with an imaging function and a radio communication function. After being swallowed by a subject (human body) from the mouth of a subject (human body) for an observation (examination), the capsule endoscope travels through body cavities, e.g., inside internal organs such as stomach and small intestine following peristaltic movements, and sequentially captures images until naturally discharged.

Image data acquired by the capsule endoscope inside the subject while the capsule endoscope travels through the body cavities is sequentially transmitted to an outside via radio communication. The transmitted data is received and stored after predetermined processing by a receiving apparatus provided outside the subject. When the subject carries and uses the receiving apparatus provided with a receiving mechanism, a signal processing_mechanism, and a storing mechanism, the subject can move freely after swallowing the capsule endoscope until discharging the same. In a conventional capsule endoscope system, after the capsule endoscope is discharged, a doctor and a nurse can display images inside the internal organs on a display unit based on the image data accumulated in a memory, and make diagnosis (See Patent Document 1).

Patent Document 1: Japanese Patent Application Laid-Open No. 2003-19111

### DISCLOSURE OF INVENTION

### PROBLEM TO BE SOLVED BY THE INVENTION

However, in the receiving apparatus provided in the conventional capsule endoscope system, an operation of the receiving mechanism and an operation of the signal processing mechanism interfere with each other. Specifically, in the receiving apparatus provided in the conventional capsule endoscope system, noises generated by the operation of one mechanism affect the operation of the other mechanism.

To alleviate such inconvenience, some propose to mount the receiving mechanism and the signal processing mechanism on separate boards, respectively, so that two mechanisms are distanced from each other by a predetermined length within the receiving apparatus in the conventional system. When such a structure is adopted, however, the number of boards incorporated in the receiving apparatus increases, thereby increasing the size of the receiving apparatus. Particularly in the capsule endoscope system, the increase in the size of the receiving apparatus, which is carried by the patient or the like, is not appropriate, since it is preferable to alleviate the physical burden on the patient or the like while the capsule endoscope is inside the body of the subject.

Other propose to cover each of the receiving mechanism and the signal processing mechanism with an electromagnetic wave shielding member. The electromagnetic wave shielding member covering each of the mechanisms prevents the noises generated by one mechanism from reaching the other mechanism, thereby preventing the interference of the operation. When such a structure is adopted, however, the structure becomes complicated. In addition, since the receiving mechanism and the signal processing mechanism are basically electrically connected with each other, complete shielding of one from the other is difficult to accomplish. Therefore, complete blocking of the noise cannot be readily achieved with such a structure.

In view of the foregoing, an object of the present invention is to provide a receiving apparatus which suppresses a mutual interference of the operation of the receiving mechanism and the operation of the signal processing mechanism while avoiding the increase in the size of the receiving apparatus, and to provide a body insertable system having the above receiving apparatus.

### MEANS FOR SOLVING PROBLEM

To solve the problems as described above and to achieve an object, a receiving apparatus according to the present invention as set forth in claim 1 is a receiving apparatus that performs reception processing on radio signals transmitted intermittently, and the receiving apparatus includes a receiving unit that performs reception processing on radio signals received by receiving antennas, and extracts original signals contained in the radio signals; a data processing unit that performs predetermined data processing on the original signals supplied from the receiving unit; and a timing controller that controls the receiving unit and the data processing unit so that the data processing unit stops the data processing while the receiving unit performs the reception processing, and the receiving unit stops the reception processing while the data processing unit performs the data processing.

According to the present invention as set forth in claim 1, since the timing controller provided controls the receiving unit and the data processing unit so that the period during which the receiving unit performs the processing operation is a different period from the period during which the data processing unit performs the processing operation, it is possible to prevent the noises generated from the processing operation of one of the receiving unit and the data processing unit from negatively affecting the processing operation of the other.

Further, in the receiving apparatus according to the present invention as recited in claim 2, the timing controller has functions of temporarily storing the original signals generated by the receiving unit, and supplying the original signals to the data processing unit when the data processing unit performs processing.

Still further, in the receiving apparatus according to the present invention as set forth in claim 3, the timing controller includes a FIFO circuit which can temporarily store input signals, a writing controller which controls the FIFO circuit so as to store signals supplied from the receiving unit when the receiving unit performs the reception processing, and a reading controller which controls the FIFO circuit so as to supply the signals stored to the data processing unit when the data processing unit performs the data processing.

Still further, in the receiving apparatus according to the present invention as recited in claim 4, the receiving unit maintains an operable state while the data processing unit performs an operation, and the data processing unit maintains an operable state while the receiving unit performs an operation, the writing controller controls the FIFO circuit so as not to store the signals supplied from the receiving unit while the data processing unit performs the data processing, and the reading controller controls the FIFO circuit so as not to supply the signals stored to the data processing unit while the receiving unit performs the reception processing.

Still further, in the receiving apparatus according to the present invention as set forth in claim 5, the radio signal contains the original signal which is based on predetermined image data, and the data processing unit reconfigures the image data based on the original signal extracted by the receiving unit.

Still further, a body insertable system according to the present invention as set forth in claim 6 includes a body insertable device that is introduced into a subject and intermittently transmits radio signals containing original signals corresponding to intra-subject information acquired, and a receiving apparatus that receives the radio signals transmitted from the body insertable device and performs predetermined processing. The body insertable device includes an intra-subject information acquiring unit that acquires intra-subject information which is information of an interior of the subject, and a transmitting unit that transmits the radio signals containing the original signals corresponding to the intra-subject information acquired by the intra-subject information acquiring unit. The receiving apparatus includes a receiving unit that performs reception processing on the radio signals received by receiving antennas, and extracts the original signals contained in the radio signals, a data processing unit that reconfigures data by performing predetermined data processing on the original signals supplied by the receiving unit, and a timing controller which controls the receiving unit and the data processing unit so that the data processing unit stops the data processing while the receiving unit performs the reception processing, and the receiving unit stops the reception processing while the data processing unit performs the data processing.

Still further, in the body insertable system according to the present invention as set forth in claim 7, the timing controller includes a FIFO circuit which can temporarily store input signals, a writing controller which controls the FIFO circuit so as to store signals supplied from the receiving unit when the receiving unit performs the reception processing, and a reading controller which controls the FIFO circuit so as to supply the signals stored to the data processing unit when the data processing unit performs the data processing.

Still further, in the body insertable system according to the present invention as set forth in claim 8, the intra-subject information acquiring unit has a function of acquiring an intra-subject image as the intra-subject information, and the data processing unit reconfigures the intra-subject image based on the original signals supplied.

### EFFECT OF THE INVENTION

The receiving apparatus and the body insertable system according to the present invention are advantageous in that the receiving apparatus and the body insertable system include the timing controller which controls the receiving unit and the data processing unit so that the receiving unit and the data processing unit perform the processing operation in different period, whereby the noises generated by the processing operation of one of the receiving unit and the data processing unit can be prevented from negatively affecting the processing operation of the other.

### BRIEF DESCRIPTION OF DRAWINGS

FIG. 1 is a schematic diagram of an overall structure of a body insertable system according to an embodiment;
FIG. 2 is a schematic block diagram of a structure of a capsule endoscope provided in the body insertable system;
FIG. 3 is a schematic block diagram of a structure of a receiving apparatus provided in the body insertable system;
FIG. 4 is a schematic timing chart of timing of processing operations of elements in the receiving apparatus; and
FIG. 5 is a schematic diagram of operations of the elements in the receiving apparatus during an image processing period.

### EXPLANATIONS OF LETTERS OR NUMERALS

- 1: Subject
- 2: Capsule endoscope
- 3: Receiving apparatus
- 4: Display device
- 5: Portable recording medium
- 6a to 6h: Receiving antenna
- 8: Intra-subject image acquiring unit
- 9: Transmitting unit
- 10: Control unit
- 11: Power supply unit
- 12: LED
- 13: LED driving circuit
- 14: CCD
- 15: CCD driving circuit
- 16: Transmitting circuit
- 17: Transmitting antenna
- 21: Antenna selector
- 22: Receiving circuit
- 23: Binarizing circuit
- 24: A/D converter
- 25: Bridge circuit
- 26: Image processing unit
- 27: Storage unit
- 28: Timing controller
- 29: Power supply unit
- 30: FIFO circuit
- 31: Writing controller
- 32: Reading controller

### BEST MODE(S) FOR CARRYING OUT THE INVENTION

Exemplary embodiments of a body insertable device and a body insertable system according to the present invention will be described below. It should be noted that the drawings are merely schematic, and the ratio between the thickness and width in each portion or the ratio of thickness of one portion to another may be different in an actual device or system. The dimensional relation and the ratio may be different from one drawing to another.

### First Embodiment

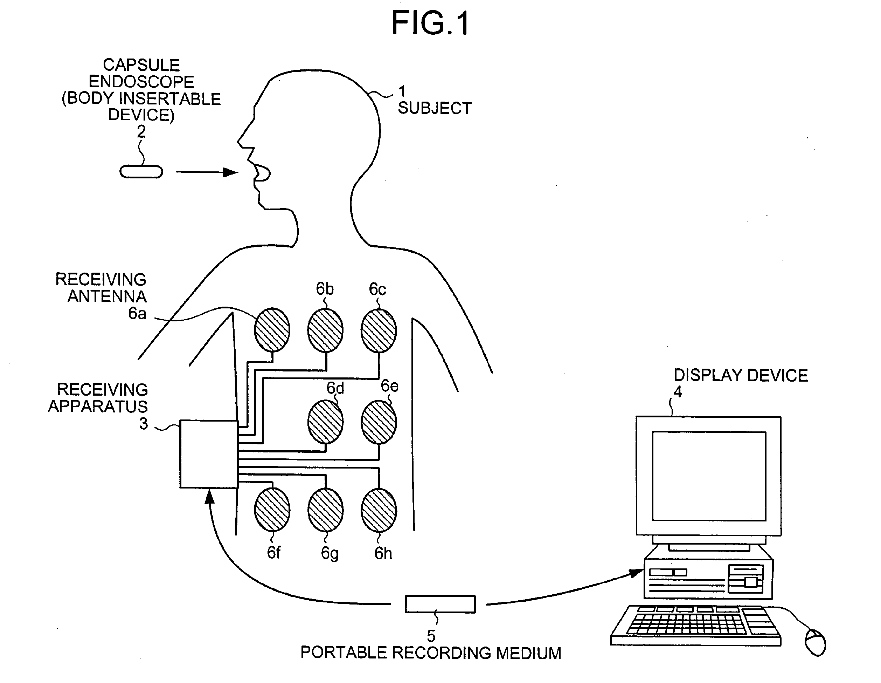

FIG. 1 is a schematic diagram of an overall structure of a body insertable system according to an embodiment. As shown in FIG. 1, the body insertable system according to the embodiment includes a capsule endoscope 2 which is introduced into a subject 1 and travels along a passage, a receiving apparatus 3 which receives radio signals transmitted from the capsule endoscope 2 and containing intra-subject information, a display device 4 which displays a content of the intra-subject information contained in the radio signals received by the receiving apparatus 3, and a portable recording medium 5 which serves for information delivery between the receiving apparatus 3 and the display device 4.

The display device 4 serves to display intra-subject images, for example, that are captured by the capsule endoscope 2 and received by the receiving apparatus 3. The display device 4 is configured like a workstation, for example, that displays images based on data obtained from the portable recording medium 5. Specifically, the display device 4 may be configured to directly display the images as in a CRT display and a liquid crystal display. Alternatively, the display device 4 may output the images or the like to other media, as in a printer.

The portable recording medium 5 can be attached to and detached from the receiving apparatus 3 and the display device 4. When the portable recording medium 5 is attached to the receiving apparatus 3 and the display device 4, information can be output from or recorded into the portable recording medium 5. Specifically, when the capsule endoscope 2 travels through body cavities of the subject 1, the portable recording medium 5 is attached to the receiving apparatus 3 and records the intra-subject images. After the capsule endoscope 2 is discharged from the subject 1, the portable recording medium 5 is removed from the receiving apparatus 3 and attached to the display device 4. Then, the display device 4 reads out the recorded data. Since the data transfer between the receiving apparatus 3 and the display device 4 is carried out with the portable recording medium 5 such as a Compact Flash (registered trademark) memory, the subject 1 can move freely even while the capsule endoscope 2 travels inside the subject 1, dissimilar to a system where the receiving apparatus 3 and the display device 4 are connected by a cable.

Receiving antennas 6a to 6h are formed with a loop antenna, for example. The loop antenna is fixed on a body surface of the subject 1 at a predetermined position during use. Each of the receiving antennas 6a to 6h preferably has a holder for securing the loop antenna on the body surface of the subject 1.

The capsule endoscope 2 will be described. The capsule endoscope 2 works as an example of a body insertable device of the present invention. The capsule endoscope 2 serves to capture the intra-subject images and to transmit radio signals that contain image data obtained via the image capturing to the receiving apparatus 3.

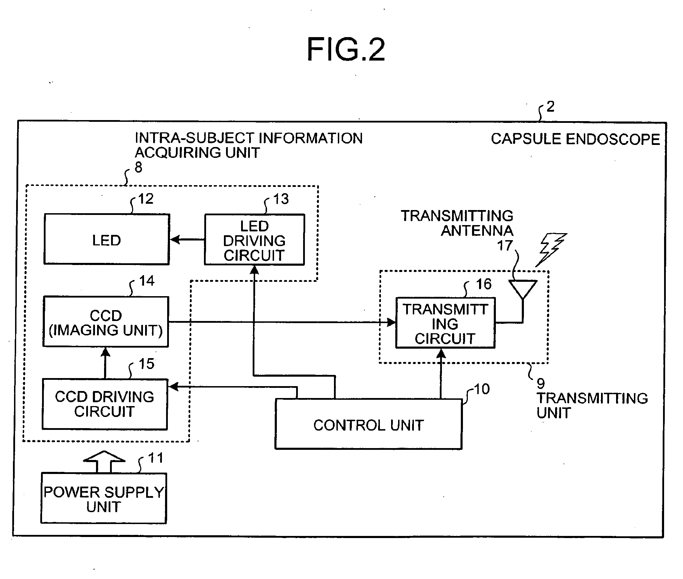

FIG. 2 is a schematic block diagram of a structure of the capsule endoscope 2. As shown in FIG. 2, the capsule endoscope 2 includes an intra-subject information acquiring unit 8 that acquires the intra-subject information, a transmitting unit 9 that radio transmits radio signals containing the intra-subject information to an outside of the subject 1, a control unit 10 that controls a driven state of each of the intra-subject information acquiring unit 8 and the transmitting unit 9, and a power supply unit 11 that supplies driving power to elements provided in the capsule endoscope 2.

The intra-subject information acquiring unit 8 serves to acquire the intra-subject images as images concerning a predetermined region inside the subject 1. Specifically, the intra-subject information acquiring unit 8 includes an LED 12 that outputs illumination light, an LED driving circuit 13 that controls a driven state of the LED 12, a CCD 14 that acquires intra-subject images, and a CCD driving circuit 15 that controls a driven state of the CCD 14.

The transmitting unit 9 serves to radio transmit the data of the intra-subject images acquired by the intra-subject information acquiring unit 8 after performing necessary processing, such as modulation thereon. Specifically, the transmitting unit 9 includes a transmitting circuit 16 that generates original signals based on input data and generates radio signals by performing processing such as modulation on the original signals, and a transmitting antenna 17 that serves to transmit the radio signals output from the transmitting circuit 16.

In the embodiment, the transmitting unit 9 does not perform the transmission operation of the radio signals constantly. Rather, the transmitting unit 9 is configured so as to intermittently transmit the radio signals by alternately repeating a transmission period and a suspension period. For the reduction of power consumption by the capsule endoscope 2, the intra-subject information acquiring unit 8 of the embodiment captures the image approximately every 0.5 second so that the amount of image data acquired by the intra-subject information acquiring unit 8 is reduced. Accordingly, the amount of transmitted data is reduced. The transmitting unit 9 performs the transmission operation for approximately 0.28 second to transmit each of the image data captured at 0.5-second intervals, and does not perform the transmission operation for the remaining approximately 0.22 second, which is the suspension period.

The control unit 10 serves to control a driven state and the like of the intra-subject information acquiring unit 8 and the like provided in the capsule endoscope 2. Specifically, the control unit 10 has a function of performing a general control of the elements, and a function of driving the intra-subject information acquiring unit 8 and the transmitting unit 9 in synchronization with each other.

Further, the control unit 10 has a function of controlling an imaging rate of the intra-subject images captured by the intra-subject information acquiring unit 8. The capsule endoscope 2 preferably continues to capture and transmit the intra-subject images for several hours, e.g., approximately eight hours, depending on the amount of power supplied from the power supply unit 11. In the embodiment, the control unit 10 controls the intra-subject information acquiring unit 8 so as to capture the intra-subject image at the imaging interval of approximately 0.5 second, so that the long-time driving of the capsule endoscope 2 can be realized through the reduction of power consumption of the capsule endoscope 2.

The power supply unit 11 serves to supply the driving power to the intra-subject information acquiring unit 8, the transmitting unit 9, and the control unit 10. In the embodiment, the power supply unit 11 is configured with a primary battery. Alternatively, however, the power supply unit 11 may be configured with a rechargeable battery, or may be configured so as to accumulate the power supplied by radio from outside.

The capsule endoscope 2 with the above described structure acquires the intra-subject images, i.e., images inside the subject 1, by the intra-subject information acquiring unit 8, and intermittently transmits the radio signals containing the acquired image data to the outside by the transmitting unit 9 after introduced into the body from the mouth of the subject 1 until discharged to the outside of the body. As described later, the body insertable system of the embodiment eliminates negative effect of the noises generated by the processing operation inside the receiving apparatus 3 by utilizing the intermittent transmission of the radio signals.

The receiving apparatus 3 will be described. The receiving apparatus 3 serves to receive the radio signals transmitted from the capsule endoscope 2 and to reconfigure the data concerning the intra-subject images contained in the radio signals.

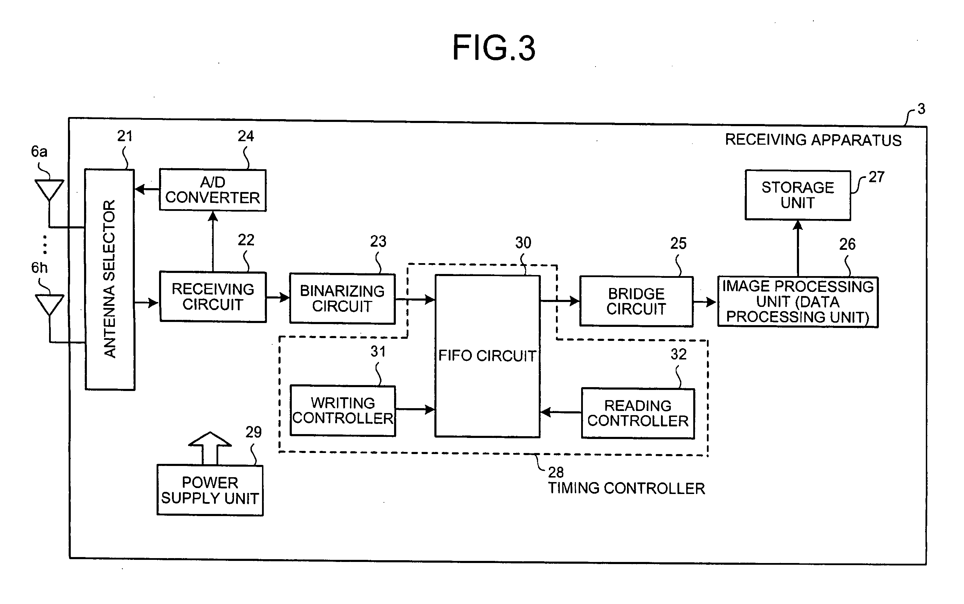

FIG. 3 is a schematic block diagram of a structure of the receiving apparatus 3. As shown in FIG. 3, the receiving apparatus 3 includes an antenna selector 21 which selects an appropriate one for the reception of the radio signals from the plural receiving antennas 6a to 6h, a receiving circuit 22 which performs processing such as demodulation on the radio signals received via the receiving antenna which is selected from the receiving antennas 66a to 6h by the antenna selector 21, a binarizing circuit 23 which converts the original signals (signals before modulation by the transmitting unit 9) extracted by the receiving circuit 22 into binarized digital signals, and an A/D converter 24 which converts a received strength signals supplied from the receiving circuit 22 into predetermined digital signals. The receiving apparatus 3 further includes an image processing unit 26 which reconfigures intra-subject image data based on the original signals that are binarized by the binarizing circuit 23 and transmitted through a bridge circuit 25, and a storage unit 27 which stores image data reconfigured by the image processing unit 26. The receiving apparatus 3 of the embodiment further includes a timing controller 28 which adjusts timing of reception processing by the receiving circuit 22 and timing of image processing by the image processing unit 26 described later, and a power supply unit 29 which supplies driving power to each element in the receiving apparatus 3.

The antenna selector 21 serves to select a most appropriate one for the reception from the plural receiving antennas 6a to 6h, and output the radio signals received by the selected receiving antenna to the receiving circuit 22. Specifically, the antenna selector 21 sequentially selects one of the receiving antennas 6a to 6h by switching and makes the selected antenna receive the radio signals in advance, and outputs the received radio signals to the receiving circuit 22. The receiving circuit 22 has a function of supplying analog signals of RSSI (Received Signal Strength Indicator) to the A/D converter 24. The A/D converter 24 converts the analog signals supplied from the receiving circuit 22 into the digital signals and outputs the resulting digital signals to the antenna selector 21. The antenna selector 21 selects the receiving antenna, the strength of the RSSI digital signal corresponding to which and supplied from the A/D converter 24 is highest, and outputs the radio signals received by the selected receiving antenna to the receiving circuit 22. Along with the movement of the capsule endoscope 2 inside the subject 1, the receiving antenna which is most appropriate for the reception changes over time. Therefore, the antenna selector 21 preferably performs an antenna selecting operation plural times.

The receiving circuit 22 serves to extract the original signals by performing processing such as demodulation on the received radio signals. In the embodiment, the receiving circuit 22 serves to extract and output the original signals in the state of analog signals. The extracted original signals are converted into digital signals by the binarizing circuit 23, and the resulting digital signals are supplied to the timing controller 28.

The image processing unit 26 serves to reconfigure the image data concerning the intra-subject images based on the original signals supplied from the binarizing circuit 23. Specifically, the image processing unit 26 is configured with an operation processing mechanism such as a Central Processing Unit (CPU) that performs a predetermined operation and a memory mechanism such as a Synchronous Dynamic Random Access Memory (SDRAM) that temporarily stores the data, for example.

The timing controller 28 serves to prevent the noises generated in the receiving circuit 22 and the image processing unit 26 from negatively affecting the operation of one another, by controlling the processing timing of each of the receiving circuit 22 and the image processing unit 26. The timing can be controlled in various manners. In the embodiment, the receiving circuit 22 and the image processing unit 26 are steadily kept in the driven state, however, the receiving circuit 22 and the image processing unit 26 are prevented from performing the processing operations simultaneously through the control of the input/output timing of the data to be processes by the receiving circuit 22 and the image processing unit 26.

Specifically, the timing controller 28 includes a FIFO circuit 30 which temporarily stores the original signals (more precisely, binarized original signals; the same applied below) extracted by the receiving circuit 22, a writing controller 31 which controls timing of data writing to the FIFO circuit 30, and a reading controller 32 which controls timing of reading out of data stored in the FIFO circuit 30.

Having the above described structure, the timing controller 28 can temporarily store the original signals extracted by the receiving circuit 22 before supplying the same to the image processing unit 26. The receiving circuit 22 and the image processing unit 26 are prevented from performing the processing operation simultaneously through an appropriate control of the writing timing and the reading timing of the data to/from the FIFO circuit 30 by the writing controller 31 and the reading controller 32, and at the same time, the noises generated from the processing operation of one of the receiving circuit 22 and the image processing unit 26 are prevented from giving negative effects on the processing operation of the other of the receiving circuit 22 and the image processing unit 26.

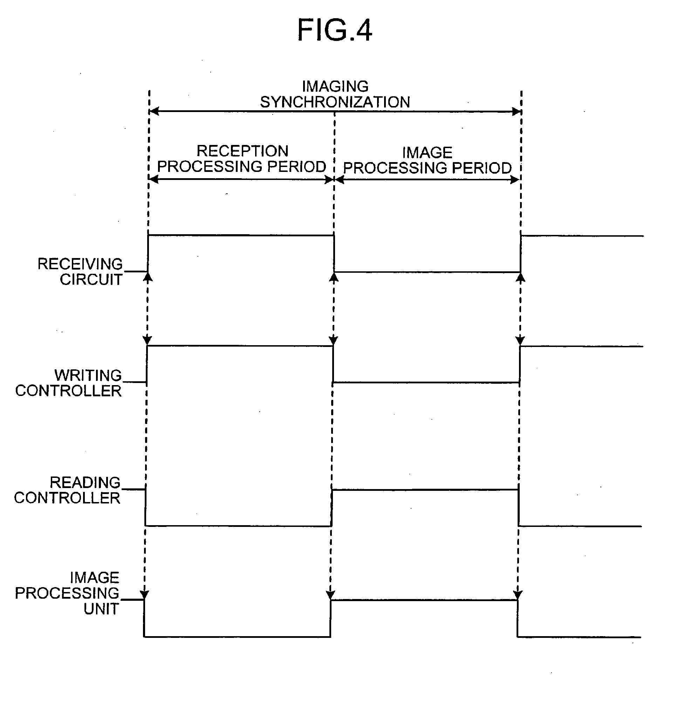

The control of the timing of the processing operations of the receiving circuit 22 and the image processing unit 26 by the timing controller 28 will be described. FIG. 4 is a timing chart of the operation timing of elements provided in the receiving apparatus 3. As shown in FIG. 4, the operation timing of the element is largely divided into a reception processing period and an image processing period. In the reception processing period and the image processing period, reception processing of extracting the original signals from the radio signals and reconfiguration processing of image data based on the original signals are performed, respectively.

In the embodiment, in the reception processing period, the receiving circuit 22 and the binarizing circuit 23 perform the processing operations, and the writing controller 31 outputs a writing command to the FIFO circuit 30. In the receiving apparatus 3 during the reception processing period, one of the receiving antennas 6a to 6h receives the radio signals, the receiving circuit 22 extracts original signals in a form of the analog signals, the binarizing circuit 23 digitizes the extracted original signals, and the FIFO circuit 30 temporarily stores the digitized original signals.

On the other hand, as is clear from FIG. 4, the reading controller 32 does not output a reading command to the FIFO circuit 30 during the reception processing period. Hence, the original signals written in the FIFO circuit 30 are not output to the bridge circuit 25, and the bridge circuit 25 and the image processing unit 26 do not perform processing. As can be seen from the foregoing, during the reception processing period, only the receiving circuit 22, the binarizing circuit 23, and the writing controller 31 perform respective processing, and thus, the writing processing of the original signal into the FIFO circuit 30 is performed.

The reception processing period is set according to the timing of transmission of the radio signals from the transmitting unit 9 provided in the capsule endoscope 2. As described above, the intra-subject information acquiring unit 8 of the capsule endoscope 2 captures the intra-subject image at approximately 0.5-second intervals, and the transmitting unit 9 has a function of sequentially transmitting the radio signals containing the intra-subject images acquired at such intervals. The transmitting unit 9 does not use all the time period of the 0.5 second for the transmission operation. Instead, the transmitting unit 9 performs the transmission operation of the radio signals over approximately a half of the 0.5-second period, for example, approximately 280-ms time period.

Thus, the reception processing period shown in FIG. 4 is set according to the timing of transmission of the radio signals from the transmitting unit 9, and more precisely, the reception processing period is set so that the reception processing period includes at least the period during which the radio signals transmitted from the transmitting unit 9 are received via the receiving antenna 6. Specifically, the reception processing period may be set by a separate mechanism provided in the receiving apparatus 3 for setting the reception processing period such that the reception processing period matches with the transmission period of the radio signals from the transmitting unit 9, at an outset of the driving of the capsule endoscope 2, for example. In the embodiment, the receiving circuit 22 is driven not only for the reception processing period, but for the image processing period, and remains capable of performing the reception processing over both the reception processing period and the image processing period. Such setting of the receiving circuit 22 may be utilized for the setting of the reception processing period. Specifically, the reception processing period may be set as a period during which the strength of the radio signals received by the receiving circuit 22 exceeds a predetermined threshold, and the writing controller 31 may give command to the FIFO circuit 30 during such a period.

Processing during the image processing period will be described. The image processing period is set so that the image processing period covers a period other than the reception processing period in the imaging period of the intra-subject information acquiring unit 8 provided in the capsule endoscope 2 as described above. As shown in FIG. 4, while the writing controller 31 and the receiving circuit 22 stop the processing, the reading controller 32 gives a reading command to the FIFO circuit 30. As a result, digital data of the original signals that are written into the FIFO circuit 30 during the reception processing period is output to the bridge circuit 25. Then, the output original signals are supplied to the image processing unit 26 via the bridge circuit 25, and the image processing unit 26 performs the image processing. Specifically, the image processing unit 26 performs a series of processing including reconfiguration of the image data concerning the intra-subject image based on the original signals and a supply of the obtained image data to the storage unit 27, during the image processing period.

Advantages of the body insertable system of the embodiment will be described. The body insertable system according to the embodiment adopts a structure in which the reception processing and the image processing are performed in a different period in the receiving apparatus 3, whereby the body insertable system of the embodiment has an advantage that the noises generated by the operation of one of the receiving circuit 22, for example, and the image processing unit 26, for example, can be prevented from negatively affecting the operation of the other of the receiving circuit 22, for example, and the image processing unit 26, for example.

As described above, in the embodiment, the radio signals from the capsule endoscope 2 are intermittently transmitted and not continuously transmitted, for the reduction of the power consumption by the capsule endoscope 2, for example. Therefore, in the receiving apparatus 3, the receiving circuit 22 or the like does not need to perform processing of the continuously received radio signals, and the receiving circuit 22 can be configured so as to perform the reception processing only during the period when the radio signals are supplied corresponding to the imaging period. Therefore, the reception processing period for extracting the original signals from the radio signals and the image processing period for reconfiguring the image data based on the original signals can be set for the processing of the receiving apparatus 3.

As shown in FIG. 4, in the receiving apparatus 3, the receiving circuit 22 (and the binarizing circuit 23 and the writing controller 31) is set so as to perform the processing only during the reception processing period, and the image processing unit 26 (and the bridge circuit 25 and the reading controller 32) is set so as to perform the processing only during the image processing period according to the operation of the timing controller 28. Therefore, even if the processing operation of one of the receiving circuit 22 and the image processing unit 26 generates noises, the other of the receiving circuit 22 and the image processing unit 26 does not perform the processing operation at the time. Thus, it is possible to prevent the noises generated by one of the receiving circuit 22 and the image processing unit 26 from negatively affecting the processing of the other.

Further, even though the FIFO circuit 30 or the like are additionally provided in the timing controller 28, the receiving apparatus does not become larger. Since the FIFO circuit 30 or the like can be made sufficiently small with the use of conventional techniques, the body insertable system of the embodiment can enjoy the advantage that the prevention of negative influence from the noises in the receiving circuit 22 and the image processing unit 26 can be realized while the increase in the size of the apparatus is suppressed.

Further, in the body insertable system of the embodiment, the timing controller 28 has the FIFO circuit 30, and is configured so as to delay the timing of the image processing from the timing of the reception processing by controlling the timing of the writing operation and the reading operation of the FIFO circuit 30 through the writing controller 31 and the reading controller 32. By adopting the above structure, the body insertable system of the embodiment has an advantage that the increase in the power consumption of the receiving apparatus 3 is suppressed while the noises generated by one element can be prevented from negatively affecting the operation of the other element.

As described above, in the embodiment, the capsule endoscope 2 is configured to intermittently transmit the radio signals in accordance with the imaging interval of approximately 0.5 second. Hence, in the receiving apparatus 3, the reception processing period and the image processing period are sequentially repeated approximately every 0.5 second. It is not appropriate to repeat ON and OFF of the receiving circuit 22 and the image processing unit 26 corresponding to the short cycle described above, in terms of reduction of power consumption and the stable operations. Therefore, in the embodiment, the receiving circuit 22 and the like receives driving power and remains in an operable state even during the image processing period during which the receiving circuit 22 or the like do not perform the processing operation.
The same applies to the image processing unit 26 and the like. The image processing unit 26 receives the driving power and remains capable of processing the input signals not only during the image processing period but also during the reception processing period during which the image processing unit 26 does not perform the processing operation.

On the other hand, when the above described structure is adopted, it is not easy to completely eliminate the effect of the noises regardless of the setting of the processing period. Hence, in the embodiment, the timing controller 28 is provided with the FIFO circuit 30 so as to eliminate the noise influence through appropriate control of the FIFO circuit 30.

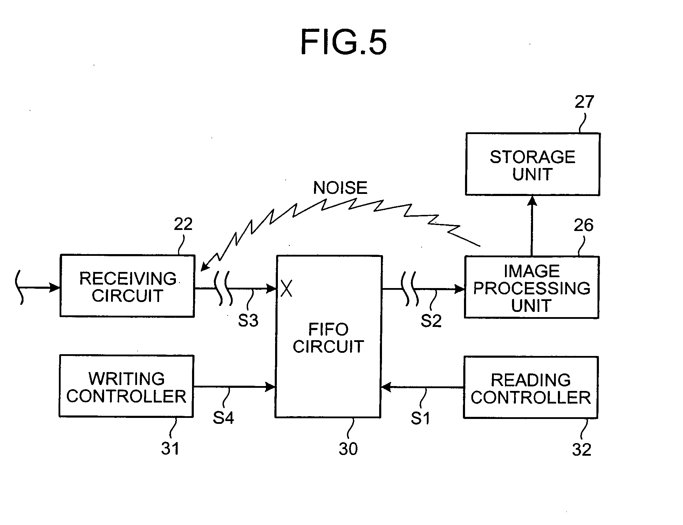

FIG. 5 is a schematic diagram showing a state of each element during the image processing period. As shown in FIG. 5, the image processing unit 26 reconfigures the image data based on the original signals S2 supplied from the FIFO circuit 30 in response to a reading control signal supplied from the reading controller 32 during the image processing period, and the noises are generated along with the processing operation. The generated noises are received, for example, by the receiving antenna 6, and supplied to the receiving circuit 22. As described above, the receiving circuit 22 receives the driving power during the image processing period and remains capable of receiving the supplied radio signals. Hence, if the receiving circuit 22 receives the noises generated in the image processing unit 26, the receiving circuit 22 ends up performing reception processing and the processed signals S3 are output from the receiving circuit 22.

In the embodiment, however, the writing controller 31 supplies a control signal S4 instructing that there should be no writing into the FIFO circuit 30 during the image processing period. Therefore, during the image processing period, the FIFO circuit 30 does not store the input signals. Hence, even if the signals S3 attributable to the noises generated along with the processing operation of the image processing unit 26 are supplied from the receiving circuit 22, the signals S3 are not stored in the FIFO circuit 30, and would not reach the image processing unit 26.

Thus, since the body insertable system according to the embodiment is provided with the FIFO circuit 30, even though the receiving circuit 22 and the image processing unit 26 remain drivable throughout the reception processing period and the image processing period, the noises generated in one element can be prevented from negatively affecting the processing operation of the other element. Thus, the receiving apparatus 3 has advantages that the increase in the power consumption can be suppressed, the stability in operation of each element can be guaranteed, and that the noises generated in one of the receiving circuit 22 and the image processing unit 26 can be prevented from negatively affecting the processing operation of the other.

In the above, the present invention is described based on the embodiment. The present invention, however, should not be interpreted as to be limited to the embodiment described above, and those skilled in the art can reach various embodiments and modifications. For example, in the embodiment, the radio signals transmitted from the capsule endoscope 2 contain the original signals corresponding to the image data, and the receiving apparatus 3 is provided with the image processing unit 26 to reconfigure the image data based on the original signals. It is obvious, however, that the present invention is applicable to a receiving apparatus which receives original signals corresponding to data other than the image data.
For example, the receiving apparatus can be configured to include a general signal processing unit in place of the image processing unit 26.

Further, in the embodiment, the timing controller 28 includes the FIFO circuit 30. Alternatively, however, the timing controller 28 may include a shift register, stack, or the like in place of the FIFO circuit 30. Even when the timing controller 28 has such an alternative structure, the advantage of the timing controller 28 provided with the FIFO circuit 30 can be obtained when other circuit or the like are combined as necessary.

Further, in the embodiment, the timing controller 28 is configured so as to prevent simultaneous processing operations through the control of the timing of data input/output of each element. Alternatively, the timing controller 28 may control the driving timing of respective elements. For example, when the timing controller 28 controls the power supply or the like so that the receiving circuit 22 is driven only during the reception period and the image processing unit 26 is driven only during the image processing period, the simultaneous processing operation can be prevented, whereby the noises of one element can be prevented from negatively affecting the operation of the other.

### INDUSTRIAL APPLICABILITY

As can be seen from the foregoing, the receiving apparatus and the body insertable system including the receiving apparatus according to the present invention are useful for a medical observation apparatus which is introduced inside a human body for an observation of an examined area, and in particular is suitable for suppressing mutual interference between an operation of a receiving mechanism and an operation of a signal processing mechanism while preventing an increase in size of the apparatus and the system.

## Claims

1. A receiving apparatus performing reception processing on radio signals transmitted intermittently, comprising:
a receiving unit that performs reception processing on radio signals received by receiving antennas, and extracts original signals contained in the radio signals;
a data processing unit that performs predetermined data processing on the original signals supplied from the receiving unit; and
a timing controller that controls the receiving unit and the data processing unit so that the data processing unit stops the data processing while the receiving unit performs the reception processing, and the receiving unit stops the reception processing while the data processing unit performs the data processing.

2. The receiving apparatus according to claim 1, wherein
the timing controller has functions of temporarily storing the original signals generated by the receiving unit, and supplying the original signals to the data processing unit when the data processing unit performs processing.

3. The receiving apparatus according to claim 1 or 2, wherein the timing controller includes
a FIFO circuit which can temporarily store input signals,
a writing controller which controls the FIFO circuit so as to store signals supplied from the receiving unit when the receiving unit performs the reception processing, and
a reading controller which controls the FIFO circuit so as to supply the signals stored to the data processing unit when the data processing unit performs the data processing.

4. The receiving apparatus according to claim 3, wherein the receiving unit maintains an operable state while the data processing unit performs an operation, and the data processing unit maintains an operable state while the receiving unit performs an operation,
the writing controller controls the FIFO circuit so as not to store the signals supplied from the receiving unit while the data processing unit performs the data processing, and
the reading controller controls the FIFO circuit so as not to supply the signals stored to the data processing unit while the receiving unit performs the reception processing.

5. The receiving apparatus according to any one of claims 1 to 4, wherein
the radio signal contains the original signal which is based on predetermined image data, and
the data processing unit reconfigures the image data based on the original signal extracted by the receiving unit.

6. A body insertable system including a body insertable device that is introduced into a subject and intermittently transmits radio signals containing original signals corresponding to intra-subject information acquired, and a receiving apparatus that receives the radio signals transmitted from the body insertable device and performs predetermined processing,
the body insertable device comprising:
an intra-subject information acquiring unit that acquires intra-subject information which is information of an interior of the subject; and
a transmitting unit that transmits the radio signals containing the original signals corresponding to the intra-subject information acquired by the intra-subject information acquiring unit, and
the receiving apparatus comprising:
a receiving unit that performs reception processing on the radio signals received by receiving antennas, and extracts the original signals contained in the radio signals;
a data processing unit that reconfigures data by performing predetermined data processing on the original signals supplied by the receiving unit; and
a timing controller which controls the receiving unit and the data processing unit so that the data processing unit stops the data processing while the receiving unit performs the reception processing, and the receiving unit stops the reception processing while the data processing unit performs the data processing.

7. The body insertable system according to claim 6, wherein the timing controller includes
a FIFO circuit which can temporarily store input signals,
a writing controller which controls the FIFO circuit so as to store signals supplied from the receiving unit when the receiving unit performs the reception processing, and
a reading controller which controls the FIFO circuit so as to supply the signals stored to the data processing unit when the data processing unit performs the data processing.

8. The body insertable system according to claim 6 or 7, wherein the intra-subject information acquiring unit has a function of acquiring an intra-subject image as the intra-subject information, and
the data processing unit reconfigures the intra-subject image based on the original signals supplied.
